# EUROPEAN PATENT SPECIFICATION

(11) **EP 1 248 591 B1**
(45) Date of publication and mention of the grant of the patent: **08.08.2007**
(21) Application number: 01900136.1
(22) Date of filing: 08.01.2001
(51) Int. Cl.: A61K 8/04, A61K 8/41, A61K 8/44, A61K 8/84, A61K 31/14, A61K 31/205, A61K 31/28, A61Q 15/00, A61Q 17/00

(54) **ANTI-MICROBIAL COMPOSITIONS COMPRISING A SALT OF A TRANSITION METAL CHELATOR**
ANTIMIKROBIELLE ZUBEREITUNG SALZ EINES ÜBERGANGMETALL-CHELATORS ENTHALTEND
COMPOSITIONS ANTIMICROBIENNES COMPRENANT UN SEL D'UN CHELATEUR DE METAL DE TRANSITION

(30) Priority: 18.01.2000 GB 0001133; 18.01.2000 GB 0001132
(43) Date of publication of application: 16.10.2002
(73) Proprietor: UNILEVER PLC, London EC4P 4BQ (GB); UNILEVER N.V., 3013 AL Rotterdam (NL)
(72) Inventor: JOHNSON, Paula Ann, Bebington Wirral Merseyside CH63 3JW (GB); LANDA, Andrew Sjaak, Bebington Wirral Merseyside CH63 3JW (GB); MAKIN, Stephen Anthony, Bebington Wirral Merseyside CH63 3JW (GB); MCMILLAN, Ian Robert, Bebington Wirral Merseyside CH63 3JW (GB)
(74) Representative: Elliott, Peter William
(86) International application number: PCT/EP2001/000118
(87) International publication number: WO 2001/052805

(56) References cited:
- DE-A- 19 620 644
- US-A- 3 507 796
- US-A- 4 356 190

## Description

### Field of Invention

This invention relates to the field of anti-microbial compositions and to methods of reducing microbial numbers. In particular, this invention is concerned with reducing microbial numbers upon the surface of the human body or in close proximity thereto and thereby reducing body malodour. The compositions and methods involved utilise particular transition metal chelator salts as anti-microbial agents. When used on the human body, the compositions and methods of the invention are of greatest benefit when used on the most malodorous areas of the human body, for example the underarm areas or feet.

### Background

Anti-microbial agents may function by a variety of means. When used upon the human body, such agents may significantly reduce microbial numbers either by reducing perspiration or by directly affecting the micro-organisms on the body surface-as represented herein by skin. It is with this latter class of agents, often called deodorant agents, that this invention is largely concerned.

Most deodorant agents reduce the number of viable micro-organisms on the surface of the skin. It is well known that sweat is usually odourless until it has been degraded by the skin microflora. Typical deodorants include ethanol and triclosan (2',4,4'-trichloro-2'-hydroxydiphenyl ether) which is a well known anti-microbial agent. However, the deodorising effect obtained with such deodorants wears off with the passage of time and the microflora progressively recover their numbers.

There is, therefore, a continuing requirement for effective and long lasting deodorant compositions on the market. The problem to be solved is not simply reducing microbial numbers on the body surface; equally important is maintaining low microbial numbers (particularly low bacterial numbers) on the body surface (particularly in the most malodorous areas, eg. the axillae).

Certain transition metal chelators have previously been incorporated into deodorant compositions. US 4,356,190 (Personal Products Co.) discloses the use of selected aminopolycarboxylic acid compounds for inhibiting the formation of short chain fatty acids by Corynebacterium on the skin surface. For topical application, alkanolamine salts are stated to be preferred. Especially preferred salts are stated to be di- and trialkanolamine salts such as triethanolamine, diethanolamine, and triisopropanolamine salts. It is also stated that solvents, including organic solvents, compatible with the system in which the chelator is incorporated may be employed.

It should be noted that the selection of counter-ions for chelators in anti-microbial compositions has a bearing on a further problem common in the field of anti-microbial compositions: that of compatibility of components and stability of products (see later).

WO 97/02010 (Procter and Gamble Co.) discloses the use of chelators selected from the succinic acid, glutaric acid, and phosphonic acid classes as bactericidal compounds. The only chelator salt actually exemplified is trisodium ethylenediamine disuccinate (Na₃EDDS).

WO 97/44006 (Ciba Speciality Chemicals Holding, Inc.) claims the use of nitrogen-containing complexing agents for the anti-microbial treatment of the skin and of textile fibre materials. Particular complexing agents-mentioned include those formed from neutralising EDDS with ethanolamine or laurylamine. Deodorant compositions comprising EDDS are also disclosed.

WO 97/01360 (Concat Ltd.) claims a method of inhibiting bacterial growth using particular substituted polyaza compounds that show affinity for first transition series elements. It is stated that compatible salts may be formed by neutralisation with inorganic or organic bases, including primary, secondary and tertiary amines, notably ethanolamine, diethanolamine, morpholine, glucamine, N,N-dimethylglucamine, and N-methylglucamine.

Other patents indicate that transition metal chelators can improve the efficacy of particular known anti-microbials. WO 89/12399 (Public Health Research Institute of the City of New York) discloses improved performance of lanthionine-containing bacteriocins in compositions also comprising a transition metal chelator. WO 97/09974 (Laboratoire Medix) discloses compositions comprising chlorhexidine and a chelator. EP 0019670 B1 (Glyco Chemicals, Inc.) discloses anti-microbial compositions comprising a condensation product of 5,5-dimethyl hydantoin and formaldehyde in combination with a water-soluble chelating agent selected from ethylenediaminetetraacetic acid (EDTA), diethylenetriaminepentaacetic acid (DTPA) or the alkali metal salts thereof. US 4,199,602 (Economics Laboratory, Inc.) discloses the potentiation of anti-microbial nitroalkanes by aminocarboxylic-type chelating agents. US 5,688,516 (University of Texas System *et al*) discloses compositions comprising non-glycopeptide anti-microbials. (other than vancomycin) in combination with a selection of components, including a chelating agent. WO 99/10017 (University of Texas System *et al*) discloses a method for controlling the growth of micro-organisms using a chelating agent and an anti-microbial agent. GB 1,420,946 (Beecham Group Ltd.) discloses that the activity of selected phenolic anti-microbials can be vastly increased by certain chelating agents, in particular the disodium salt of EDTA.

Chelators have also been disclosed as formulation aids in cosmetic stick compositions. US 5,798,094 (Gillette Company) discloses the use of 0.3 to 1.6% of an alkali metal salt of a chelating agent in cosmetic sticks to help achieve clarity; US 5,516,511 (Procter and Gamble Co.) discloses particular antiperspirant gel compositions in which chelators are used during manufacture to prevent reaction between the active and the primary gellant; and US 5,849,276 (Procter and Gamble Co.) mentions chelants in antiperspirant stick compositions, although such materials are stated to be optional "non-active" components.

### Summary of the Invention

This invention is concerned with the amelioration of the two problems of anti-microbial compositions alluded to above: the problem of obtaining prolonged anti-microbial activity, together with the problem of obtaining compatibility of components and the stability of products.

It has now been discovered that anti-microbial compositions comprising certain transition metal chelator salts give prolonged anti-microbial activity, for example lasting a day, and greater compatibility with other common components of anti-microbial compositions, in particular organic solvents like ethanol. The prolonged anti-microbial activity often manifests itself as a long-lasting deodorancy benefit. The greater compatibility of the chelator salts of the invention leads to greater formulation flexibility; for example, homogeneous solutions can generally be made in a range of organic solvents, in at least some solutions homogeneity being maintained even in the presence of other additional highly hydrophobic components, even at elevated pressure, as found in aerosol products. Organic solutions of the chelator salts of the invention offer advantages with respect to many of the problems associated with alternative suspension products, for example valve blocking, settling and caking of the suspended solids, and uneven application to the surface requiring treatment.

An additional benefit of the anti-microbialcompositions of the invention is that they can, if desired, be formulated with relatively low levels of water. This can be.of value in compositions applied to the human body, as compositions containing relatively high levels of water can sometimes cause an undesirable wet sensation on application. It can also be of benefit with regard to container choice: low water content compositions enable metal containers to be used with less risk of corrosion. A further benefit of compositions having low water levels is their compatibility with additional hydrophobic components, for example perfume components (see "Perfumery: practice and principles", R.R.Calkin and S.Jellinek, [Wiley, 1994, p171]).

Thus, according to a first aspect of the present invention, there is provided an anti-microbial composition for use on the outer surface of human body or on apparel worn in close proximity thereto comprising a carrier material and a salt of a transition metal chelator comprising a transition metal chelator anion and an organic cation, characterised in that the cation comprises a protonated or quaternised amine, other than triisopropanolamine, containing 0 to 3 hydroxyl groups per N-substituent and at least one N-substituent comprising a C₁-C₁₀ terminal hydrocarbyl group.

Herein, hydroxyl groups are any O-H groups present in the organic cation and hydrocarbyl groups are radicals containing solely carbon and hydrogen atoms.

According to a related second aspect of the present invention, there is provided an anti-microbial composition comprising a solution in an organic solvent of a salt of a transition metal chelator comprising an anionic chelator for a transition metal and an organic cation, characterised in that the cation comprises a protonated or quaternised amine, other than triisopropanolamine, containing 0 to 3 hydroxyl groups per N-substituent and at least one N-substituent comprising a C₁-C₁₀ terminal hydrocarbyl group. In preferred embodiments, such anti-microbial compositions function as deodorant compositions; in other preferred embodiments less than 50% by weight of water is present in the composition, excluding any volatile propellant that may be present.

According to a third aspect of the present invention, there is provided a cosmetic method of controlling microbial numbers, said method comprising the application to the outer surface of the human body or to apparel worn in close proximity thereto of an anti-microbial composition comprising a carrier material and a salt of a transition metal chelator comprising a transition metal chelator anion and an organic cation, characterised in that the cation comprises a protonated or quaternised amine, other than triisopropanolamine, containing 0 to 3 hydroxyl groups per N-substituent and at least one N-substituent comprising a C₁-C₁₀ terminal hydrocarbyl group. A particular embodiment of this aspect of the invention comprises the application of an anti-microbial composition comprising a solution in an organic solvent of the transition metal chelator salt. An application of this aspect of the invention is the control of microbial numbers on the outer surface of the human body, for example the skin. Such application may represent a method of inhibiting the generation of human body malodour. This method may also be used to deliver enhanced fragrance intensity from a fragrance-containing composition according to the invention.

According to a fourth aspect of the present invention, there is provided a method for the manufacture of an anti-microbial composition, said method comprising the formation of a solution in an organic solvent of a salt of a transition metal chelator salt comprising a transition metal chelator anion and an organic cation, characterised in that the cation comprises a protonated or quaternised amine, other than triisopropanolamine, containing 0 to 3 hydroxyl groups per N-substituent and at least one N-substituent comprising a C₁-C₁₀ terminal hydrocarbyl group.

### Detailed Description

The novel anti-microbial compositions of the invention perform unexpectedly well in terms of anti-microbial efficacy and maintenance of low malodour, particularly when applied to the human body. Without wishing to be bound by theory, it is hypothesised that after reduction of microbial numbers by other co-applied agents and/or by some external treatment like washing, the chelator salt effectively inhibits the up-take of essential transition metal ion nutrients by the remaining microbes, thereby minimising their re-growth. In addition, the invention offers significant advantages in terms of compatibility of components and product stability. The chelator salts described are readily incorporated into organic solvents and give extensive compatibility with other components. Without wishing to be bound by theory, it is hypothesised that the chelator salt cation is required to be relatively hydrophobic in order to counter-balance the hydrophilic nature of the chelator salt anion. Hence, the chelator salt cations comprise only limited hydroxyl groups and comprise at least one C₁-C₁₀ terminal hydrocarbyl group.

Preferred compositions of the invention are homogeneous solutions. Such solution compositions have advantages with respect to many of the problems associated with alternative suspension compositions; for example, valve blocking, settling and caking of the suspended solids, and uneven application can all be reduced.

When the invention takes the form of a solution of the chelator salt in an organic solvent, it is advantageous that the selected chelator salt and the organic solvent are highly compatible. Such solutions of chelator salt in organic solvent are preferably of a concentration of 0.5% to 5% by weight. It is preferred that the salt is soluble in the organic solvent in the presence of less than 10% by weight of water in the solvent system, more preferably in the presence of less than 5%_by weight of water, and most preferably in the presence of less than 1% by weight of water in the solvent system.

The chelator salt may be present in compositions of the invention in any form. For example, the chelator salt may be diluted with a volatile propellant and used as an aerosol; with a liquid and used, for example, as a roll-on or squeeze-spray product; or with a thickener or structurant and used, for example, as a cream, gel or solid stick product.

The compositions of the invention are intended for application to the outer surface of the human body, in particular to the skin, or to apparel worn in close proximity thereto, such as shoes, socks, or undergarments. The former mode of application excludes use within body cavities such as the mouth. The latter mode of application excludes use of laundry cleaning compositions.

The compositions of the invention may be applied to the surface requiring treatment by any means. For example, liquid products might be absorbed onto a carrier matrix like paper, fabric, or sponge and applied by contacting said carrier matrix with the surface. Solid or semi-solid products might be applied by direct contact or might be dissolved or dispersed in a liquid medium prior to application.

### Chelators

Preferred transition metal chelator salts possess anions having affinity for iron (III), preferably high affinity for iron (III); that is to say, a binding constant for iron (III) of greater than 10¹⁰, or, for optimum performance; greater than 10²⁶. The 'iron (III) binding constant' referred to above is the absolute stability constant for the chelator-iron (III) complex. Such values are independent of pH and are measured on the most anionic, fully deprotonated form of the chelator. Measurements can be made potentiometrically, and in a number of other ways. Full details of suitable methods can be found in " Determination and Use of Stability Constants" , A. E. Martell and R. J. Motekaitis (VCH, New York, 1989). Tables of applicable values may be found in numerous sources, for example " Critical Stability Constants" , R. M. Smith and A. E. Martell (Plenum Pub. Corp., 1977).

Preferred chelator salts are formed from chelators which are able to significantly inhibit the growth of a relevant micro-organism when present, in a medium containing said micro-organism, at a concentration of 3 x 10⁻⁶ mol.dm⁻³ or less. Inhibition is considered significant when growth of the relevant micro-organism on a supporting medium can be reduced by at least 30%, preferably by at least 45%. When the surface to be treated is human skin, a relevant micro-organism is *Staphlococcus epidermidis* and chelators capable of achieving significant inhibition include diethylenetriaminepentaacetic acid (DTPA) and triethylenetetraaminehexaacetic acid (TTHA), but exclude ethylenediaminetetraacetic acid (EDTA) and trans-1,2-diaminocyclohexane-N,N,N',N'-tetraacetic acid (CDTA).

The chelators used in the present invention preferably have acid forms with at least two, preferably at least four, and most preferably at least five ionisable acid groups. The acid groups are preferably carboxylic and/or phosphonic, but may be sulphonic or phosphinic, or any mixture of these groups.

Preferred chelators with phosphonic acid groups are, in the acid form, diethylenetriaminepenta(methylphosphonic) acid (DTPMP), ethanehydroxydiphosphonic acid (EHDP), ethylenediaminetetra(methylenephosphonic acid) (EDTMP), and hexamethylenediaminetetra(methylenephosphonic acid) (HMDTMP).

Particularly suitable chelators with acid forms having carboxylic acid groups are polycarboxylate compounds, in particular aminopolycarboxylate compounds. The acid forms of the aminopolycarboxylate compounds include EDTA, CDTA, and ethylenediaminedisuccinic acid (EDDS). More preferred aminopolycarboxylate chelators have the acid forms DTPA, TTHA, and N,N'-ethylenebis[2-(2-hydroxyphenyl)glycine] (EDDHA).

The chelator salts preferably have only moderate molecular weight, by which it is meant that the chelators, in their acid forms, have a molecular weight of less than 1000, more preferably 200 to 800, and most preferably 290 to 580, and in their salt form have a molecular weight of less than 2000, more preferably 300 to 1400, and most preferably 500 to 1000.

The chelator salt is preferably incorporated into the composition at a level of 0.01% to 10%, more preferably at a level of 0.05% to 5%, and most preferably at a level 0.3% to 3% by weight of the composition, excluding any volatile propellant present. Mixtures of chelator salts may also be used.

### Cations

Preferred transition metal chelators have cations of formula R¹R²R³R⁴ N⁽⁺⁾, wherein R¹ is H or CH₃; R², R³, and R⁴ are each independently H or an aliphatic or aromatic substituent containing 0 to 3 hydroxyl groups, optionally interrupted and/or substituted by functional groups such as ether, amine, ester, or amide; with the provisos that at least one of R², R³, or R⁴ comprises a C₁-C₁₀ terminal hydrocarbyl group, optionally R² and R³ together forming a ring as the terminal hydrocarbyl group, and that R², R³, and R⁴ are not all CH₂CH (OH) CH₃ groups.

Particularly preferred cations have at least one of R², R³, or R⁴ comprising an H atom directly attached to an N atom or an O atom. The presence of an H atom directly attached to an O atom (ie. a hydroxyl group) in at least one of R², R³, or R⁴ is especially preferred, up to the aforementioned limit of 3 hydroxyl groups per N-substituent.

Other particularly preferred transition metal chelator salts have cations comprising N-substituents (R¹, R², R³, and R⁴, according to the formula) that collectively contain a total of 0 to 3 hydroxyl groups, preferably 0 to 2 hydroxyl groups.

In many desirable cations, each N-substituent (R¹, R², R³, and R⁴, according to the formula) contains not more:than one hydroxyl group.

Preferred cations are those derived from aliphatic amines, in particular, aliphatic amines in which the ratio of the total number of H atoms directly attached to an N atom or an O atom to the total number of carbon atoms is not greater than 3:4.

It is advantageous that the cations are relatively hydrophobic, as defined by their limited hydroxyl group content and their possession of at least one C₁-C₁₀ terminal hydrocarbyl group. Examples of suitable terminal hydrocarbyl groups include C₁-C₆ alkyl and cycloalkyl (where possible). Preferred terminal hydrocarbyl groups are methyl, ethyl, propyl, and cyclohexyl.

Partial salts of chelator acids possessing more than one acidic group may also be employed; such salts retain one or more non-ionised acid groups. It is preferred that the chelator salts of such acids have at least 40%, more preferably at least 60%, of their available acid groups in the form of salts with a protonated or quaternised amine, other than triisopropanolamine, containing 0 to 3 hydroxyl groups per N-substituent and at least one N-substituent comprising a C₁-C₁₀ terminal hydrocarbyl group. Also claimed are salts where the cations are in part protonated or quaternised amines and in part some other cation, for example an alkali metal cation, in particular a sodium ion.

Preferably, the amount of amine added is that which would lead to an aqueous solution of the chelator salt having a pH of between 6 and 8 (at a molar concentration of chelator salt equal to that present in the composition).

Preferred chelator salts have protonated amines as cations. The following further preferences apply to the amine used:
1. That the amine is of relatively low odour. This is of potential benefit during manufacture and during selection and use of compositions comprising such amine salts.
   Related to this point is the preference for the amine to have relatively low volatility: a boiling point of 130°C or greater at atmospheric pressure being preferred.
2. That the amine is of low melting point (30°C or less). This can be of advantage with regard to formulation and processing.

Preferred chelator salts are salts of isopropanolamine, 2-amino-2-ethyl-1,3-propanediol, 2-(N,N-dimethylamino)-2-methyl-1-propanol (DMAMP) and N,N-dimethylaminoethanol. Particularly preferred chelator salts are salts of 2-amino-2-methyl-1-propanol (AMP), diisopropanolamine, 2-aminobutan-1-ol, and cyclohexylamine, diisopropylamine, tert-butylamine, N,N-diethylhexylamine, and mixtures thereof.

Another group of preferred chelator salts comprise cations derived from amines that are relatively hydrophobic, lacking N-H bonds and/or O-H bonds. Such amines could alternatively be described as non-hydroxylated and/or tertiary amines. Particular examples include diisopropylamine, N,N-diethylhexylamine, *tert*-butylamine, DMAMP, and cyclohexylamine. These amines enable stable chelator salt compositions to be formed at particularly low ratios of water to other liquids present (for example, less than 5:95 by weight) and/or at particularly high levels of volatile propellant (for example, greater than 40% or 50% by weight of the composition); indeed, homogeneous solutions may be formed at these compositions.

### Carrier Material

A carrier material for the chelator salt is an essential component in compositions of the invention. The carrier material may be hydrophobic or hydrophilic, solid or liquid. Suitable solid carrier materials include talcs and other inert powders. Preferred carrier materials are liquids. Hydrophobic liquids suitable for use with the chelator salts of the invention include liquid silicones, that is to say, liquid polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202. Alternatively, non-silicone hydrophobic liquids may be used. Such materials include mineral oils, hydrogenated polyisobutene, polydecene, paraffins, isoparaffins of at least 10 carbon atoms, and aliphatic or aromatic ester oils (eg. isopropyl myristate, lauryl myristate, isopropyl palmitate, diisopropyl sebecate, diisopropyl adipate, or C₈ to C₁₈ alkyl benzoates)-Hydrophilic liquid carrier materials, for example water, may also be employed.

Particularly preferred liquid carrier materials comprise organic solvents. To aid compatibility between the chelator salt and the organic solvent, especially preferred organic solvents are relatively hydrophilic, having a c.logP of less than 2, especially -2 to 1, and in particular -0.5 to 0.5. c.logP is the calculated logarithm to the base 10 of the octanol:water partition coefficient; a method for calculating such values may be found in " Computer-assisted computation of partition coefficients from molecular structures using fragment constants" , J.Chou and P.Jurs, J. Chem. Inf. Comput. Sci., 19, 172 (1979). In addition, preferred organic solvents have a melting point of less than 10°C, preferably less than 5°C; this can benefit both low temperature storage stability and ease of manufacture. A class of preferred organic solvents are aliphatic alcohols (monohydric or polyhydric, preferably having 2 to 8 carbon atoms) and polyglycol ethers, preferably oligoglycol ethers having only 2 to 5 repeat units. Examples include dipropylene glycol, glycerol (c.logP -1.538) propylene glycol (c.logP -1.06.), butylene glycol (c.logP -0.728), ethanol (c.logP 0.235), propanol (c.logP 0.294), isopropanol (c.logP -0.074), and industrial methylated spirits. The most preferred organic solvents are aliphatic alcohols, in particular those having 2 to 3 carbon atoms, especially ethanol and isopropanol.

Mixtures of carrier materials may also be used. The amount of carrier material employed is preferably from 30% to 99%, more preferably 60% to 98%, expressed as a weight percentage of the total weight of non-volatile components of the composition.

When organic solvent is present in the composition, it is preferably present at from 30% to 98% by weight of the total weight of the liquid components of the composition; more preferably the organic solvent comprises from 60% to 97% by weight of the total liquids present (excluding any liquified volatile propellant present).

For some applications, it is desired that less than 50% by weight of water is present as part of the liquid components of the composition. Indeed, the benefits of the invention are particularly great when the ratio of other liquid components to water is greater than 50:50, more particularly when this ratio is greater than 65:35, and especially when this ratio is greater than 90:10. For some preferred compositions, the ratio of other liquid components to water is between 95:5 and 99:1, by weight.

Liquid components in the context of this invention are those having a melting point of less than 20°C at atmospheric pressure.

Preferred compositions with an organic solvent comprise a solution of the chelator salt in said organic solvent. Such solutions are preferably homogeneous, preferably having an absorbance, relative to the solvent, of less than 0.2, especially less than 0.1 (for a 1 cm pathlength at 600 nm) measured using a Pharmacia Biotech Ultrospec 200 Spectrophotometer or similar instrument.

### Additional Components

An additional component that can sometimes augment the efficacy of a composition is an additional anti-microbial agent. Such anti-microbial agents are typically applied in a preceding step or simultaneously and serve to lower the viable microbial population of the surface treated. Most of the classes of agents commonly used in the art can be incorporated into compositions of the invention. Levels of incorporation are preferably from 0.01% to 3%, more preferably from 0.03% to 0.5% by weight of the composition, excluding any volatile propellant present. Preferred compositions of the invention comprise an additional anti-microbial agent having a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of a relevant micro-organism is observed, relative to a control medium without an anti-microbial agent, over a period of 16 to 24 hours at 37°C. The "relevant micro-organism" used for testing should be representative of those associated with the substrate to be treated. When the substrate to be treated is human skin, a relevant micro-organism is *Staphylococcus epidermidis.* Other relevant micro-organisms include *Coryneform spp., Salmonella spp., Escherichia Coli,* and *Pseudomonas spp.,* in particular *P. aeruginosa.* Details of suitable methods for determining MICs can be found in " Antimicrobial Agents and Susceptibility Testing" , C.Thornsberry, (in " Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Preferred additional anti-microbials agents are cationic bactericides, in particular organic cationic bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in " Deodorant Ingredients", S.A.Makin and M.R.Lowry, in " Antiperspirants and Deodorants" , Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ^{™} available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05-0.3%; and 3,7,11-trimethyldodeca-2,6,10-trienol (farnesol), preferably used at up to 1% by weight of the composition and more preferably at up to 0.5%.

In a related aspect, good anti-microbial and/or deodorancy benefits are obtained from an anti-microbial product comprising a transition metal chelator having any organic counter-ion and a further organic anti-microbial agent having a'minimum inhibitory concentration of 1 mg.ml⁻¹ or less. In this related aspect, the organic counter-ion of the chelator is not limited as hereinbefore described; although organic counter-ions limited in such a manner do represent a preferred option. The preferences described above for the further/additional organic anti-microbial agent and the transition metal chelator salt apply equally to this related aspect. Similarly, a carrier material as described hereinbefore and further additional components as described hereinafter may also be employed in this related aspect. A further feature of this related aspect is that it is not essential that the chelator salt and the further organic anti-microbial agent are part of the same composition. The anti-microbial benefit derived may be gained by independent application of the chelator salt and the further organic anti-microbial agent. Such application may be concurrent or consecutive, provided that the treated substrate experiences the presence of both components at the same time. When the components are applied from independent compositions, it is preferred that the product also comprises a means for, and/or instruction for, both of the compositions to be applied to the substrate requiring treatment. It is preferred that the anti-microbial product comprises a transition metal chelator and a further organic anti-microbial agent that are both present in the same composition. The benefits found with such compositions can include good product aesthetics, lack of product separation, attainment of desirable rheology, visco-stability, and good dispensing.

Inorganic anti-microbial agents may also be used in the compositions of the invention. Such materials can often also function as anti-perspirant agents when present at a suitable concentration. Examples are often selected from astringent active salts, including, in particular, aluminium, zirconium and mixed aluminium/ zirconium salts, including both inorganic salts, salts with organic anions and complexes. Preferred astringent salts include aluminium, zirconium and aluminium/zirconium halides and halohydrate salts, such as chlorohydrates. When included, preferred levels of incorporation are from 0.5% to 60%, particularly from 5% to 30% or 40% and especially from 5% or 10% to 30% or 35% by weight of the composition. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

It should be noted that incorporation of amphoteric or cationic anti-microbial agents makes it particularly important to use the chelator salts in accord with the present invention. This is particularly true of organic anti-microbial agents, of cationic anti-microbial agents, and especially true of organic polycationic anti-microbial agents. In this context, " polycationic" means possessing more than one positive charge, although the importance of the use of chelator salts in accord with the present invention is even greater in the presence of organic polycationic anti-microbial agents that possess more than five positive charges per molecule.

Phenolic anti-oxidants can also augment the efficacy of compositions of the invention. Preferred materials are butylated hydroxytoluene (BHT) and butylated hydroxyanisole (BHA). Such agents are preferably used at 0.05% to 5%, more preferably 0.075% to 2.5%, and most preferably 0.1% to 1% by weight of the composition, excluding any volatile propellant present.

Structurants and emulsifiers are further additional components of the compositions of the invention that are highly desirable in certain product forms. Structurants, when employed, are preferably present at from 1% to 30% by weight of the composition, whilst emulsifiers are preferably present at from 0.1% to 10% by weight of the composition. In roll-ons, such materials help control the rate at which product is dispensed by the roll ball. In stick compositions, such materials can form gels or solids from solutions or suspensions of the chelator salt in a carrier fluid. Suitable structurants for use in such compositions of the invention include cellulosic thickeners such as hydroxy propyl cellulose and hydroxy ethyl cellulose, and dibenzylidene sorbitol. Emulsion pump sprays, roll-ons, creams, and gel compositions according to the invention can be formed using a range of oils, waxes, and emulsifiers. Suitable emulsifiers include steareth-2, steareth-20, steareth-21, ceteareth-20, glyceryl stearate, cetyl alcohol, cetearyl alcohol, PEG-20 stearate, and dimethicone copolyol. Suspension aerosols, roll-ons, sticks, and creams require structurants to slow sedimentation (in fluid compositions) and to give the desired product consistency to non-fluid compositions. Suitable structurants include sodium stearate, stearyl alcohol, cetyl alcohol, hydrogenated castor oil, synthetic waxes, paraffin waxes, hydroxystearic acid, dibutyl lauroyl glutamide, alkyl silicone waxes, quaternium-18 bentonite, quaternium-18 hectorite, silica, and propylene carbonate. Some of the above materials also function as suspending agents in certain compositions.

Further emulsifiers desirable in certain compositions of the invention are perfume solubilisers and wash-off agents. Examples of the'former include PEG-hydrogenated castor oil, available from BASF in the Cremaphor RH and CO ranges, preferably present at up to 1.5% by weight, more preferably 0.3 to 0.7% by weight. Examples of the latter include poly(oxyethylene) ethers.

Certain sensory modifiers are further desirable components in the compositions of the invention. Such materials are preferably used at a level of up to 20% by weight of the composition. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely-divided silica (eg. Aerosil 200), polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

Fragrance is also a desirable additional component in the compositions of the invention. Suitable materials include conventional perfumes, such as perfume oils and also include so-called deo-perfumes, as described in EP 545,556 and other publications. Levels of incorporation are preferably up to 4% by weight, particularly from 0.1% to 2% by weight, and especially from 0.7% to 1.7% by weight of the composition.

It should be noted that certain components of compositions perform more than one function. Such components are particularly preferred additional ingredients, their use often saving both money and formulation space. Examples of such components include ethanol, isopropyl myristate, and the many components that can act as both structurants and sensory modifiers, for example silica.

Further additional components that may also be included are colourants and preservatives, for example C₁-C₃ alkyl parabens.

### Product Forms

The compositions of the invention may take any form. Examples include wax-based sticks, soap-based sticks, compressed powder sticks, roll-on suspensions or solutions, emulsions, gels, creams, squeeze sprays, pump sprays, and aerosols. Each product form contains its own selection of additional components, some essential and some optional. The types of components typical for each of the above product forms may be incorporated in the corresponding compositions of the invention. Roll-on compositions particularly suited to the invention are simple solutions in organic solvents, although water can be tolerated in such compositions. In addition, emulsion compositions, for example oil-in-water and water-in-oil emulsions, are not excluded. Stick compositions of the invention are preferably based on either a monohydric or polyhydric alcohol organic solvent base. They are often gelled with sodium stearate, although dibenzylidene sorbitol (DBS) may alternatively be used, preferably in combination with hydroxypropyl cellulose..

### Aerosol Compositions

In one especially desirable aspect of the present invention, the chelator salt is dissolved in an organic solvent and diluted with a volatile propellant to form a homogeneous pressurised aerosol composition. Such compositions are very difficult to achieve without the use of the particular chelator salts of the invention - stability and compatibility of components must be maintained at elevated pressure in the presence of an often highly hydrophobic volatile propellant. Preferred anti-microbial aerosol compositions comprise an organic solution of a chelator salt according to the invention in combination with a non-chlorinated volatile propellant. Particularly preferred variants of such compositions comprise an organic solvent having a c.logP of less than 2 and are homogeneous pressurised solutions, preferably having an absorbance, relative to the solvent, of less than 0.2, especially less than 0.1 (for a 1 cm pathlength at 600 nm) measured using a Pharmacia Biotech Ultrospec 200 Spectrophotometer or similar instrument.

The aerosol composition may comprise from 30 to 99 parts by weight, and particularly 30 to 60 parts by weight of propellant and the remainder (respectively 70 to 1 and particularly 70 to 40 parts by weight) of the deodorant base composition.

The propellant may be selected from liquified hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquified hydrocarbon gases, and especially C₃ to C₆ hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane.

Other propellants that can be contemplated include alkyl ethers, such as dimethyl ether or compressed non-reactive gasses such air, nitrogen or carbon dioxide.

The base composition, which is mixed with the propellant, may comprise any of the following components as preferred additional ingredients: an organic solvent of c.logP less than 2 (eg. ethanol), a fragrance, or an emollient/co-solvent (eg. isopropyl myristate or propylene glycol).

The aerosol formulation can incorporate, if desired, anticlogging agents in conventional amounts, in order to prevent or minimise the occurrence of solid occlusions in the spray nozzle.

The aerosol composition is usually filled into an aerosol canister that is capable of withstanding pressures generated by the formulation, employing conventional filling apparatus and conditions. The canister can conveniently be a metal canister commercially available fitted with a dip tube, valve and spray nozzle through which the formulation is dispensed.

### Methods of Manufacture

Manufacture of compositions of the invention typically comprises one of two possible methods: one may neutralise or part-neutralise an acidic transition metal chelator with an amine, and then introduce the chelator-amine salt so formed into a suitable carrier material; or, alternatively, one may perform an in situ neutralisation or part-neutralisation of an acidic transition metal chelator with an amine, in a suitable carrier fluid, for example an organic solvent.
Such a neutralisation or part-neutralisation can be performed by addition of the base to the acid or *vice-versa.* In a preferred method of manufacture, an acidic transition metal chelator is neutralised or part-neutralised with an amine in aqueous solution and the aqueous solution so formed is then diluted with an organic solvent, such as an alcohol. The resulting solution may then be incorporated into a product using the appropriate additive(s); for example, addition of a liquified volatile propellant to give an aerosol product.

### Examples

### (Note that "letter" codes refer to Comparative Examples.)

### Example 1: Preparation of a DTPA-AMP Aerosol Deodorant

0.52 g of DTPA was added as a powder to 65.91 g of 96% (w/w) ethanol. To this mixture was added (dropwise, with stirring) 0.38 g of AMP. The resulting mixture was stirred, with gentle heating (50°C) for 30 minutes. 0.34 g of isopropyl myristate was added to the resulting solution and mixed in. The resulting mixture was sealed into a conventional aluminium deodorant can, having valve access, and 36 g (±0.2 g) of liquefied propellant (CAP 40, *ex* Calor) was introduced into the can from a propellant 'transfer can', *via* the valve, using a polyethylene transfer device. Finally, the can was fitted with a suitable actuator to enable effective spray application of the product.

### Deodorancy Test 1

An anti-microbial composition according to the current invention (Example 1) and a control composition (Comparative Example A - lacking the chelator-amine salt, see Table 1 for compositions) were prepared according to the method described. The deodorancy performances of the two compositions were tested according to the following protocol. The results, presented in Table 1, illustrate the deodorancy benefit obtained from using a chelator salt according to the invention. This benefit is a direct result of the anti-microbial performance of the composition.

### Deodorancy protocol

The panel employed comprised 50 individuals who had been instructed to use control ethanolic deodorant products during the week prior to the test. At the start of the test, panellists were washed with unfragranced soap and test product (1.20g) applied to one axilla and control product applied (1.20g) to the other. (Product application was randomised to take into account any left/right bias). Panellists were instructed not to consume spicy food or alcohol, and not to wash under their own axillae, during the duration of the test. At least three expert assessors determined the intensity of axillary odour at 5 hours and 24 hours after application, scoring the intensity on a scale of 1-5. After each 24 hour assessment, the panellists were re-washed, and products re-applied, as above. The procedure was repeated 4 times. At the end of the test the data were analysed using standard statistical techniques.

**Table 1: DTPA-AMP salt vs. Control**

| **Component** | | **Example A** | **Example 1** |
|---|---|---|---|
| DTPA¹ (as free acid) | | 0 | 0.5 |
| AMP² | | 0 | 0.37 |
| Isopropyl myristate³ | | 0.33 | 0.33 |
| CAP40⁴ | | 35 | 35 |
| Ethanol (96%) | | to 100 | to 100 |
| | | | |
| Mean malodour intensity⁵ | 5 hour | 2.2 | 1.86 |
| | 24 hour | 2.36 | 2.01 |

All components are expressed as weight per cent of the total components added.
1. diethylenetriaminepentaacetic acid.
2. 2-amino-2-methyl-1-propanol, used to form the amine salt of the chelator.
3. Emollient.
4. Propellant, proprietary mix of butane, isobutane and propane, ex. Calor.
5. The malodour differences between the compositions were significant at the 99% level, after both 5 hours and 24 hours. (Minimum differences required for significance at the 95% and 99% confidence levels were:
   after 5 hours: 0.14 for 95% level; 0.19 for 99% level;
   after 24 hours: 0.17 for 95% level; 0.22 for 99% level).

### Anti-microbial Test 1

Example 2, indicated in Table 2, was prepared in a similar manner to Example 1 and was subjected to the following in vivo test for anti-microbial activity, together with comparative Example A.

The panel employed comprised 27 males who had been instructed to use control ethanolic deodorant products during the week prior to the test. During the first week of the test, panellists' axillae were washed each morning with unfragranced soap and no deodorant products were applied. During the second week of the test, the wash procedure was followed by the application of test product (1.20g) to one axilla and control product (1.20g) to the other. (Product application was randomised to take into account any left/right bias). Panellists were instructed not to consume spicy food or alcohol, and not to wash under their own axillae, during the duration of the test.

During the second week, samples of axillary microflora were extracted from each of the panellists immediately before the morning wash (on one of the weekdays other than the first). The axillary microflora were extracted by washing with a phosphate buffer. The extract was subjected to serial dilution and plating on selective media. This enabled the determination of the number colony forming units (CFU) of Coryneform bacteria, Staphylococci bacteria, and total aerobic bacteria per square cm of axillary skin. At the end of the test, the data were analysed using standard statistical techniques.

**Table 2: Anti-microbial Results**

| **Component** | **Example A** | **Example 2** |
|---|---|---|
| DTPA (as free acid) | 0 | 0.5 |
| AMP | 0 | 0.38 |
| Isopropyl myristate | 0.33 | 0.33 |
| Butylated hydroxytolunene | 0 | 0.10 |
| CAP40 | 35 | 35 |
| Ethanol (96%) | to 100 | to 100 |

| **Results** | **(log₁₀CFU) cm⁻²** | |
|---|---|---|
| *Staphylococci spp.* | **5.63** | **4.29** |
| *Coryneform spp.* | **4.64** | **3.46** |
| Total Aerobic bacteria | **5.68** | **4.36** |

All components are expressed as weight per cent of the total components added.

These results illustrate the anti-microbial benefit of compositions according to the invention. Each of the reductions in bacterial numbers was significant at the 99% level. (The *Staphylococci* result was significant at the 99.9% level.)

These results illustrate the anti-microbial benefit of compositions according to the invention. The reduction in the numbers of *Staphylococci* and *Coryneform* bacteria were significant at the 99.9% level.

### Deodorancy Test 2

The deodorancy protocol described above was also used to test the performance of Examples B and 3 (see Table 3). These Examples were prepared in a similar manner to Examples A and 1, with the modification that a fragrance material was added to the compositions shortly before introduction into the conventional aluminium deodorant cans.

**Table 3: Fragranced DTPA-AMP salt vs. Fragranced Control**

| **Component** | | **Example B** | **Example 3** |
|---|---|---|---|
| DTPA (as free acid) | | 0 | 0.5 |
| AMP | | 0 | 0.37 |
| Isopropyl myristate | | 0.33 | 0.33 |
| Water | | 2.53 | 2.49 |
| Ethanol | | 60.64 | 59.81 |
| CAP40 | | 35 | 35 |
| Fragrance | | 1.5 | 1.5 |
| Mean malodour intensity | 5 hour | 1.34 | 1.13 |
| | 24 hour | 2.07 | 1.71 |

All components are expressed as weight per cent of the total components added.

The malodour differences between the compositions were significant at the 99% level, after both 5 hours and 24 hours. (Minimum differences required for significance at the 95% and 99% confidence levels were:
after 5 hours: 0.10 for 95% level; 0.13 for 99% level;
after 24 hours: 0.10 for 95% level; 0.13 for 99% level).

### Solubility Tests

The following experiments illustrate the improved compatibility between chelator salts according to the invention and organic solvents.

Various salts of diethylenetriaminepentaacetic acid (DTPA) were formed by neutralising (ie. taking to pH 7.0) 30 g of DTPA suspended in water with the indicated bases to give a final volume of solution of 100 ml. The concentrated aqueous solutions were then diluted to 25 mmol.dm⁻³ DTPA using varying amounts of water and/or ethanol. Recorded in Table 4A below are the maximum concentrations of ethanol (in the final solution) that maintained a clear 25 mmol.dm⁻³ solution of DTPA.

A further test was performed on the neutral DTPA salts having an ethanol tolerance of 96% or greater, by weight. 76 mmol.kg⁻¹ solutions of these salts in 96:4 (w/w) ethanol/water, also containing perfume (1.5% w/w) and isopropyl myristate (0.33% w/w), were pressurised to about 2.7 bar with a proprietary mixture of propane, isobutane, and N-butane (22:24:54, ex Calor). The resulting pressurised systems, contained liquified propellant:base in the weight ratio 35:65, DTPA being present at about 13 mmol.kg⁻¹, based on the total weight of all components present, including the propellants. The results are also indicated in Table 4A:

**Table 4A**

| **Example** | Base | Ethanol compatibility (% v/v) | Effect of pressure |
|---|---|---|---|
| **C** | NaOH | 75 | not investigated |
| **D** | ethanolamine | 84 | not investigated |
| **E** | diethanolamine | 87 | not investigated |
| **F** | triethanolamine | 84 | not investigated |
| **G** | 2-amino-2-hydroxymethyl-1,3-propanediol (tromethamine) | 76 | not investigated |
| **H** | bis-hydroxyethyl-tromethamine | 77 | not investigated |
| **4** | isopropanolamine | > 97 | precipitated out when pressurised |
| **5** | 2-amino-2-ethyl-1,3-propanediol | > 97 | precipitated out when pressurised |
| **6** | diisopropanolamine | > 97 | no precipitation |
| **7** | 2-amino-2-methyl-1-propanol (AMP) | > 97 | no precipitation |
| **8** | 2-amino-2-butanol | > 97 | no precipitation |
| **9** | cyclohexylamine | > 97 | no precipitation |

The above results show that DTPA salts according to the invention are highly compatible with the organic solvent ethanol. These results also show that organic solvent/chelator salts according the invention can be formulated in the absence of substantial levels of water. Further, these results show that the preferred chelator salts formed from DTPA and diisopropanolamine, 2-amino-2-methyl-1-propanol (AMP), 2-aminobutan-1-ol, and cyclohexylamine, have high ethanol compatible and the ability to be formulated in compositions containing a low level of water, even at elevated pressure with hydrophobic propellant present.

In a second series of experiments, various salts of diethylenetriaminepenta(methylphosphonic) acid (DTPMP, *ex* Solutia Europe S.A.) were formed by neutralising the concentrated aqueous solution obtained from the supplier (ca. 50% w/v) with the bases indicated in Table 4B. Portions of the resulting solutions, which were ca. 440 mmole.dm⁻³ in DTPMP salt, were diluted with aqueous alcohol (various ratios) to give a concentration of 22 mmole.dm⁻³ DTPMP salt. Recorded in Table 4B are the maximum concentrations of ethanol that maintained a clear solution at this concentration.

A similar series of experiments were performed with 1-hydroxyethylidenenediphosphonic acid (HEDP, *ex* Fluka Chemical Co.). 48 mmole of this acid were dissolved in distilled water and the pH adjusted to neutrality with the bases indicated in Table 3B, to give a 970 mmol.dm⁻³ solutions of HEDP, present with varying counter-ions. Dilutions of this solution were made such that 48.5 mmol.dm⁻³ solutions were produced with varying ethanol:water ratios. The maximum concentrations of ethanol that maintained a clear solution at this concentration are recorded in Table 4B.

**Table 4B**

| **Example** | Base | Acid | EtOH compatibility (% v/v) |
|---|---|---|---|
| **I J** | NaOH | DTPMP | 32-34 |
| | | HEDP | 45-47 |
| **K L** | TEA¹ | DTPMP | 71-73 |
| | | HEDP | 82-83 |
| **10** | AMP | DTPMP | greater than 95 |
| **11** | | HEDP | greater than 97.5 |

| | | | |
|---|---|---|---|
| 1. triethanolamine. | | | |

For the sodium salts, an optically clear solution could be achieved in aqueous ethanol solutions containing only very limited ethanol levels. Above this level opaque precipitates formed which were unstable and rapidly settled, forming a second phase. This was also true for the triethanolamine salts, although the ethanol concentrations achievable were somewhat higher with this base. For the AMP salts, however, optically clear solutions were maintained even up to the maximum levels of ethanol tested. No signs of precipitate formation were observed.

In a third series of experiments, various salts of ethylenediaminetetraacetic acid (EDTA, *ex* Sigma) were formed by combining 100 mmole of EDTA with 280 mmole of the bases indicated in Table 4C, in 50 ml of 95% isopropanol. The resulting samples were magnetically stirred for 30 minutes at ambient temperature. After this time any solids present were removed by filtration, dried, and weighed. Recorded in Table 3C are the amounts of solids present, expressed as a percentage of the weight of EDTA initially present.

**Table 4C**

| **Example** | Base | Solids present (% w/w) |
|---|---|---|
| **M** | triisopropanolamine | 29.8 |
| **12** | AMP | 4.2 |
| **13** | 2-amino-1-butanol | 1.2 |
| **14** | cyclohexylamine | 1.1 |

These results indicate that EDTA salt solutions in isopropanol can be formed much more effectively with AMP, 2-amino-1-butanol, and cyclohexylamine, than with triisopropanolamine.

A similar series of experiments were performed using 95% ethanol as the solvent. The results, presented in Table 4D, show the benefit of isopropanolamine and diisopropanolamine over triisopropanolamine.

**Table 4D**

| **Example** | Base | Solids present (% w/w) |
|---|---|---|
| **N** | triisopropanolamine | 65.0 |
| **15** | diisopropanolamine | 1.4 |
| **16** | isopropanolamine | 1.4 |

### Benefits with Additional Anti-microbial Agent

The following experiments were performed to illustrate the improved deodorancy performance of compositions comprising chelator-amine salts of the invention and an additional cationic anti-microbial agent. The performance of the compositions was assessed using deodorancy tests performed in accordance with the protocol described under " Deodorancy Test 1", with the amendment that products were dosed as roll-ons, with a dosage of 0.3 g per application.

Comparative Example P (see Table 4A) was prepared in the following manner. 1.0 g of DTPA (as the free acid) was added to 30 g of water. The pH was adjusted to about 7.0 by dropwise addition of 1M sodium hydroxide solution. 0.5 g of a 20%(w/v) aqueous solution of poly(hexamethylenebiguanide) chloride (PHMBC) was then added to this solution. 0.65 g of hydroxypropylcellulose (HPC) was added to 60 g of ethanol whilst shearing at a speed of about 8000 rpm on a Silverson L4RT mixer (ex. Silverson, Chesham, Bucks.). A homogenous solution was obtained, which was allowed to cool to ambient temperature. 1.5 g of fragrance oil was then added with stirring. The ethanolic HPC solution was then mixed with the aqueous solution of DTPA and the total weight adjusted to 100g with water.

Comparative Example O (see Table 5A) was prepared in a similar manner, with the omission of the DTPA and sodium hydroxide solution.

**Table 5A: PHMBC vs. PHMBC/DTPA (sodium salt)**

| **Component** | | **Example O** | **Example P** |
|---|---|---|---|
| PHMBC¹ | | 0.1 | 0.1 |
| Na₃DTPA² | | 0 | 1.15 |
| Ethanol | | 60 | 60 |
| HPC³ | | 0.65 | 0.65 |
| Fragrance | | 1.5 | 1.5 |
| Water | | to 100 | to 100 |
| | | | |
| Mean malodour intensity⁴ | 5 hour | 1.38 | 1.44 |
| | 24 hour | 1.86 | 2.05 |

All components are expressed as weight per cent of the total composition.
1. Poly(hexamethylenebiguanide) chloride, Cosmocil CQ ex Zeneca PLC.
2. DTPA trisodium salt, prepared as in Example 2.
3. Hydroxypropylcellulose, Klucel, ex Hercules.
4. The malodour difference between the compositions was significant at the 95% level after 24 hours. (Minimum differences required for significance at the 95%-and 99% confidence levels were:
   after 5 hours: 0.16 for 95% level; 0.21 for 99% level;
   after 24 hours: 0.15 for 95% level; 0.20 for 99% level).

The results in Table 5A indicate that addition of DTPA trisodium salt to a composition also comprising PHMBC and ethanol leads to a poorer deodorancy performance.

Example 17 (see table 5B) was prepared in the following manner. 1.0 g of DTPA (as the free acid) was added to 30 g of water. The pH was adjusted to about 7.0 by dropwise addition of AMP.

0.65 g of HPC and 0.043 g of poly(hexamethylenebiguanide) stearate (PHMBS, as described in WO98/56252 [Unilever PLC and NV]) was added to 60 g of ethanol whilst shearing at a speed of about 8000 rpm on a Silverson L4RT mixer (ex. Silverson, Chesham, Bucks.) A homogenous solution was obtained, which was allowed to cool. The ethanolic HPC solution was then mixed with the aqueous solution of DTPA and the total weight adjusted to 100g with water.

Comparative Example Q (see table 5B) was prepared in a similar manner, with the omission of the DTPA and AMP.

**Table 5B: PHMBS vs. PHMBS/DTPA (AMP salt)**

| **Component** | | **Example Q** | **Example 17** |
|---|---|---|---|
| PHMBS | | 0.043 | 0.043 |
| DTPA | | 0 | 1.0 |
| AMP | | 0 | 0.8 |
| Ethanol | | 60 | 60 |
| HPC | | 0.65 | 0.65 |
| Water | | to 100 | to 100 |
| | | | |
| Mean malodour intensity | 5 hour | 1.94 | 1.75 |
| | 24 hour | 2.09 | 1.92 |

All components are expressed as weight per cent of the total components added. The malodour differences between the compositions were significant at the 99% level, after both 5 hours and 24 hours. (Minimum differences required for significance at the 95% and 99% confidence levels were:
after 5 hours: 0.10 for 95% level; 0.13 for 99% level;
after 24 hours: 0.09 for 95% level; 0.12 for 99% level).

The results in Table 5B indicate that addition of DTPA/AMP salt to a deodorant composition also comprising ethanol and PHMBS leads to an improved deodorancy performance. The improved deodorancy benefit is the result of an improved anti-microbial benefit.

It should also be noted that the above benefit for the DTPA/AMP salt was present even after 24 hours, indicating prolonged maintenance of malodour reduction, a direct result of the prolonged anti-microbial activity of the composition.

### Examples 18 to 24: Aerosol Compositions with Chelator Salts of Hydrophobic Amines

The compositions indicated in Table 6 were prepared in the following manner.

For each Example, DTPA (2.00g) was added as a powder to demineralised water (2.40g). To each mixture, the indicated organic amine(s) was added, drop-wise with stirring. The weight in grams of organic amine(s) added was four times the weight percentage indicated in Table 6. The resulting mixtures were each made up to 20g with anhydrous ethanol and stirred until a homogeneous solution was obtained.

Independently, for each Example, a solution of anhydrous ethanol (30g), isospropyl myristate (1g) and butylated hydroxytoluene (0.1g) was prepared. For each Example, this solution was mixed with 5g of the appropriate amine-containing solution. To each mixture was then added fragrance (1.5g) and anhydrous ethanol (up to 45g). The resulting 45g base compositions were made into aerosol products by the addition of 55g of CAP40, using the same technique as described for Example 1.

**Table 6: Aerosol Compositions**

| **Component** | **Example** | | | | | | |
|---|---|---|---|---|---|---|---|
| | **18** | **19** | **20** | **21** | **22** | **23** | **24** |
| DTPA | 0.5 | 0.5 | 0.5 | 0.5 | 0.5 | 0.5 | 0.5 |
| BHT | 0.1 | 0.1 | 0.1 | 0.1 | 0.1 | 0.1 | 0.1 |
| Fragrance | 1.5 | 1.5 | 1.5 | 1.5 | 1.5 | 1.5 | 1.5 |
| AMP | 0 | 0.25 | 0 | 0 | 0.09 | 0 | 0 |
| DMAMP¹ | 0.49 | 0 | 0 | 0 | 0 | 0 | 0 |
| CHA² | 0 | 0.20 | 0.42 | 0 | 0 | 0 | 0 |
| DIPA³ | 0 | 0 | 0 | 0.41 | 0.32 | 0 | 0 |
| t-BA⁴ | 0 | 0 | 0 | 0 | 0 | 0.31 | 0 |
| DEHA⁵ | 0 | 0 | 0 | 0 | 0 | 0 | 0.54 |
| IPM⁶ | 1.00 | 1.00 | 1.00 | 1.00 | 1.00 | 1.00 | 1.00 |
| Water | 0.6 | 0.6 | 0.6 | 0.6 | 0.6 | 0.6 | 0.6 |
| CAP40 | 55 | 55 | 55 | 55 | 55 | 55 | 55 |
| Ethanol | to 100 | to 100 | to 100 | to 100 | to 100 | to 100 | to 100 |

| | | | | | | | |
|---|---|---|---|---|---|---|---|
| All components are expressed as weight per cent of the total components added. 1. 2-(N,N-dimethylamino)-2-methyl-1-propanol. 2. Cyclohexylamine. 3. Diisopropylamine. 4. Tert-butylamine. 5. N,N-diethylhexylamine. 6. Isopropyl myristate. | | | | | | | |

All the above compositions were homogeneous solutions.
These results illustrate the successful use of non-hydroxylated or tertiary amines (ie. amines free of any O-H or N-H bonds) in compositions having low ratios of water to other liquids present and at high levels of volatile propellant.

### Tetraalkylammonium-DTPA Aerosol Compositions

The tetraalkylammonium-DTPA salt compositions indicated in Table 7 were prepared in a similar manner to Examples 18 to 24. The indicated tetraalkylammonium hydroxide salts were used, instead of the amines of Examples 18 to 24, to form the DTPA salts according to following equation:

3.3R₄N⁺ OH⁻ + X(CH₂COOH)₅ → X(CH₂COOH)_{1.7}(CH₂COO⁻ R₄N⁺)_{3.3} + 3.3H₂O

where R is methyl, ethyl, or n-butyl and X is the DTPA backbone group which links the acetate groups.

**Table 7: Tetrabutylammonium-DTPA Aerosol Composition**

| **Component** | **Example 25** | **Example 26** | **Example 27** |
|---|---|---|---|
| DTPA (as free acid) | 0.5 | 0.5 | 0.5 |
| Me₄N⁺ OH⁻ | 0.38 | | 0 |
| Et₄N⁺ OH⁻ | 0 | 0.62 | 0 |
| Bu₄N⁺ OH⁻ | 0 | 0 | 1.09 |
| IPM | 1.0 | 1.0 | 1.0 |
| Water¹ | 1.15 | 1.15 | 1.63 |
| CAP40 | 55 | 55 | 55 |
| Fragrance | 1.5 | 1.5 | 1.5 |
| BHT | 0.1 | 0.1 | 0.1 |
| Ethanol | to 100 | to 100 | to 100 |

All components are expressed as weight per cent of the total components added.
1. the water level excludes that formed from the reaction between the DPTA and the tetraalkylammonium hydroxide.

## Claims

1. An anti-microbial composition for use on the outer surface of the human body or on apparel worn in close proximity thereto comprising a carrier material and a salt of a transition metal chelator comprising a transition metal chelator anion and an organic cation, **characterised in that** the cation comprises a protonated or quaternised amine, other than triisopropanolamine, containing 0 to 3 hydroxyl groups per N-substituent and at least one N-substituent comprising a C₁-C₁₀ terminal hydrocarbyl group.

2. An anti-microbial composition according to claim 1, wherein the carrier material comprises an organic solvent.

3. An anti-microbial composition according to claim 2, comprising a solution in an organic solvent of the transition metal chelator salt.

4. An anti-microbial composition according to any of the preceding claims, that is a deodorant composition for use on the human body or in close proximity thereto.

5. An anti-microbial composition according-to any of the preceding claims, **characterised in that** the cation of the chelator salt is a protonated amine.

6. An anti-microbial composition according to claim 5, **characterised in that** the cation of the chelator salt is protonated 2-amino-2-methyl-l-propanol, cyclohexylamine, diisopropanolamine, or 2-aminobutan-1-ol.

7. An anti-microbial composition according to any of the preceding claims, **characterised in that** the organic cation is present at a level sufficient to neutralise at least 60% of any acid groups on the acid form of the chelator anion.

8. An anti-microbial composition according to any of the preceding claims, **characterised in that** the organic cation is present at a level sufficient to lead to an aqueous solution of the chelator salt having a pH of between 6 and 8 (at a molar concentration of chelator salt equal to that present in the composition).

9. An anti-microbial composition according to any of the preceding claims, **characterised in that** the anion of the transition metal chelator salt has affinity for iron (III).

10. An anti-microbial composition according to claim 8, **characterised in that** the anion of the transition metal chelator salt has a binding coefficient for iron (III) of greater than 10²⁶.

11. An anti-microbial composition according to any of the preceding claims, **characterised in that** the transition metal chelator salt is a polyaminocarboxylic acid salt.

12. An anti-microbial composition according to any of the preceding claims, **characterised in that** the anion of the transition metal chelator salt has an acid form comprising at least five acid groups.

13. An anti-microbial composition according to claim 12, **characterised in that** the transition metal chelator salt is a diethylenetriaminepentaacetic acid salt.

14. An anti-microbial composition according to any of the preceding claims, **characterised in that** less than 50% by weight of water is present in the composition, excluding any volatile propellant that may be present.

15. An anti-microbial composition according to claim 14, **characterised in that** the ratio of other liquid components to water is greater than 65:35 by weight.

16. An anti-microbial composition according to any of the preceding claims, **characterised in that** the chelator salt is present at a concentration of 0.01% to 10% by weight, excluding any volatile propellant present.

17. An anti-microbial composition according to any of the preceding claims, which is in the form of an aerosol composition comprising a volatile propellant.

18. An anti-microbial aerosol composition according to claim 16, comprising an organic solvent of c.logP less than 2 and a non-chlorinated volatile propellant, said composition being a homogeneous pressurised solution.

19. An anti-microbial composition according to any of the preceding claims, comprising an additional anti-microbial agent.

20. An anti-microbial composition according to claim 19, **characterised in that** the additional anti-microbial agent is a cationic bactericide.

21. An anti-microbial composition according to claim 20, **characterised in that** the additional anti-microbial agent is an organic cationic bactericide.

22. An anti-microbial composition according to any of the preceding claims, comprising fragrance material at up to 4% by weight of the composition.

23. A cosmetic method of controlling microbial numbers on the outer surface of the human body or on apparel worn in close proximity thereto, said method comprising the application to the outer surface of the human body or to apparel worn in close proximity thereto of an anti-microbial composition according to any of the preceding claims.

24. A cosmetic method of inhibiting the generation of human body odour, said method comprising the application to the outer surface of the human body or to apparel worn in close proximity thereto of an anti-microbial composition according to any of the claims 1 to 22.

25. A cosmetic method of delivering enhanced fragrance intensity comprising the topical application to the outer surface of the human body or to apparel worn in close proximity thereto of a composition according to claim 22.

26. A method according to any of claims 23 to 25 in which, in a preceding step, the outer surface of the human body or apparel worn in close proximity thereto is washed and/or in a preceding or simultaneous step is contacted with an anti-microbial agent thereby lowering the viable microbial population.

27. A method for the manufacture of an anti-microbial composition, said method comprising the formation of a solution in an organic solvent of a transition metal chelator salt according to claim 3.

28. A method for the manufacture of an anti-microbial composition according to claim 27, comprising the addition of an acidic chelator and an amine to water to form an aqueous solution, followed by dilution with an alcohol to form an aqueous alcohol solution, optionally followed by pressurisation with a liquified volatile propellant.

## Patentansprüche

1. Antimikrobielle Zusammensetzung zur Verwendung auf der äußeren Oberfläche des menschlichen Körpers oder auf Kleidungsstücken, die in unmittelbarer Nähe davon getragen werden, umfassend ein Trägermaterial und ein Salz eines Übergangsmetall-Chelators, das ein Übergangsmetall-Chelatoranion und ein organisches Kation umfasst, **dadurch gekennzeichnet, dass** das Kation ein protoniertes oder quaternisiertes Amin außer Triisopropanolamin enthält, das 0 bis 3 Hydroxylgruppen pro N-Substituent und mindestens einen N-Substituenten mit einer endständigen C₁-C₁₀-Hydrocarbylgruppe aufweist.

2. Antimikrobielle Zusammensetzung nach Anspruch 1, wobei das Trägermaterial ein organisches Lösungsmittel umfasst.

3. Antimikrobielle Zusammensetzung nach Anspruch 2, das eine Lösung in einem organischen Lösungsmittel des Übergangsmetall-Chelatorsalzes umfasst.

4. Antimikrobielle Zusammensetzung nach einem der vorstehenden Ansprüche, bei der es sich um eine Deodorantzusammensetzung zur Verwendung am menschlichen Körper oder in unmittelbarer Nähe davon handelt.

5. Antimikrobielle Zusammensetzung nach einem der vorstehenden Ansprüche, **dadurch gekennzeichnet, dass** das Kation des Chelatorsalzes ein protoniertes Amin ist.

6. Antimikrobielle Zusammensetzung nach Anspruch 5, **dadurch gekennzeichnet, dass** das Kation des Chelatorsalzes protoniertes 2-Amino-2-methyl-1-propanol, Cyclohexylamin, Diisopropanolamin oder 2-Aminobutan-1-ol ist.

7. Antimikrobielle Zusammensetzung nach einem der vorstehenden Ansprüche, **dadurch gekennzeichnet, dass** das organische Kation in ausreichender Menge vorhanden ist, um mindestens 60 % aller Säuregruppen auf der Säureform des Chelatoranions zu neutralisieren.

8. Antimikrobielle Zusammensetzung nach einem der vorstehenden Ansprüche, **dadurch gekennzeichnet, dass** das organische Kation in ausreichender Menge vorhanden ist, um eine wässerigen Lösung des Chelatorsalzes mit einem pH zwischen 6 und 8 zu ergeben (bei einer Molkonzentration des Chelatorsalzes, die der in der Zusammensetzung vorliegenden entspricht).

9. Antimikrobielle Zusammensetzung nach einem der vorstehenden Ansprüche, **dadurch gekennzeichnet, dass** das Anion des Übergangsmetall-Chelatorsalzes Affinität für Eisen(III) aufweist.

10. Antimikrobielle Zusammensetzung nach Anspruch 8, **dadurch gekennzeichnet, dass** das Anion des Übergangsmetall-Chelatorsalzes einen Bindungskoeffizienten für Eisen(III) von mehr als 10²⁶ hat.

11. Antimikrobielle Zusammensetzung nach einem der vorstehenden Ansprüche, **dadurch gekennzeichnet, dass** das Übergangsmetall-Chelatorsalz ein Polyaminocarbonsäuresalz ist.

12. Antimikrobielle Zusammensetzung nach einem der vorstehenden Ansprüche, **dadurch gekennzeichnet, dass** das Anion des Übergangsmetall-Chelatorsalzes eine mindestens fünf Säuregruppen umfassende Säureform hat.

13. Antimikrobielle Zusammensetzung nach Anspruch 12, **dadurch gekennzeichnet, dass** das Übergangsmetall-Chelatorsalz ein Diethylentriaminpentaessigsäuresalz ist.

14. Antimikrobielle Zusammensetzung nach einem der vorstehenden Ansprüche, **dadurch gekennzeichnet, dass** ausschließlich eines eventuell vorliegenden flüchtigen Treibmittels weniger als 50 Gew.-% Wasser in der Zusammensetzung vorhanden sind.

15. Antimikrobielle Zusammensetzung nach Anspruch 14, **dadurch gekennzeichnet, dass** das Verhältnis der anderen flüssigen Komponenten zu Wasser nach Gewicht größer als 65 : 35 ist.

16. Antimikrobielle Zusammensetzung nach einem der vorstehenden Ansprüche, **dadurch gekennzeichnet, dass** das Chelatorsalz ausschließlich eines eventuell vorhandenen flüchtigen Treibmittels in einer Konzentration von 0,01 bis 10 Gew.-% vorhanden ist.

17. Antimikrobielle Zusammensetzung nach einem der vorstehenden Ansprüche, die in Form einer ein flüchtiges Treibmittel umfassenden Aerosolzusammensetzung vorliegt.

18. Antimikrobielle Zusammensetzung nach Anspruch 16, die ein organisches Lösungsmittel mit einem c.logP von weniger als 2 und ein nicht chloriertes flüchtiges Treibmittel umfasst, wobei es sich bei der Zusammensetzung um eine homogene unter Druck gesetzte Lösung handelt.

19. Antimikrobielle Zusammensetzung nach einem der vorstehenden Ansprüche, die ein zusätzliches antimikrobielles Mittel umfasst.

20. Antimikrobielle Zusammensetzung nach Anspruch 19, **dadurch gekennzeichnet, dass** das zusätzliche antimikrobielle Mittel ein kationisches Bakterizid ist.

21. Antimikrobielle Zusammensetzung nach Anspruch 20, **dadurch gekennzeichnet, dass** das zusätzliche antimikrobielle Mittel ein organisches kationisches Bakterizid ist.

22. Antimikrobielle Zusammensetzung nach einem der vorstehenden Ansprüche, die Duftstoffe bis zu 4 Gew.-% der Zusammensetzung enthält.

23. Kosmetisches Verfahren zur Steuerung der Mikrobenzahl auf der äußeren Oberfläche des menschlichen Körpers oder eines in unmittelbarer Nähe davon getragenen Kleidungsstücks, wobei das Verfahren das Aufbringen einer antimikrobiellen Zusammensetzung nach einem der vorstehenden Ansprüche auf die äußere Oberfläche des menschlichen Körpers oder eines in unmittelbarer Nähe davon getragenen Kleidungsstücks umfasst.

24. Kosmetisches Verfahren zur Hemmung des Entstehens von Körpergeruch beim Menschen, wobei das Verfahren das Aufbringen einer antimikrobiellen Zusammensetzung nach einem der Ansprüche 1 bis 22 auf die äußere Oberfläche des menschlichen Körpers oder eines in unmittelbarer Nähe davon getragenen Kleidungsstücks umfasst.

25. Kosmetisches Verfahren zur Abgabe eines Dufts mit verstärkter Intensität, das die topische Anwendung einer Zusammensetzung nach Anspruch 22 auf die äußere Oberfläche des menschlichen Körpers oder ein in unmittelbarer Nähe davon getragenen Kleidungsstücks umfasst.

26. Verfahren nach einem der Ansprüche 23 bis 25, bei dem in einem vorgeschalteten Schritt die äußere Oberfläche des menschlichen Körpers oder eines in unmittelbarer Nähe davon getragenen Kleidungsstücks gewaschen wird und/oder in einem vorhergehenden oder gleichzeitigen Schritt mit einem antimikrobiellen Mittel in Kontakt gebracht wird, wodurch die lebensfähige Mikrobenpopulation verringert wird.

27. Verfahren zur Herstellung einer antimikrobiellen Zusammensetzung, wobei das Verfahren die Herstellung einer Lösung eines Übergangsmetall-Chelatorsalzes nach Anspruch 3 in einem organischen Lösungsmittel umfasst.

28. Verfahren zur Herstellung einer antimikrobiellen Zusammensetzung nach Anspruch 27, das die Zugabe eines sauren Chelators und eines Amins zu Wasser umfasst, um eine wässerige Lösung zu bilden, gefolgt von der Verdünnung mit einem Alkohol, um eine wässerige Alkohollösung zu bilden, ggfs. gefolgt von Unter-Druck-Setzen mit einem verflüssigten flüchtigen Treibmittel.

## Revendications

1. Composition antimicrobienne pour une utilisation sur la surface externe du corps humain ou sur un vêtement porté très proche de celui-ci, comprenant un matériau support et un sel d'un chélateur de métal de transition comprenant un anion de chélateur de métal de transition et un cation organique, **caractérisée en ce que** le cation comprend une amine protonée ou quaternisée, autre qu'une triisopropanolamine, contenant de 0 à 3 groupes hydroxyles par substituant N et au moins un substituant N comprenant un groupe hydrocarbyle terminal en C₁-C₁₀.

2. Composition antimicrobienne selon la revendication 1, dans laquelle le matériau support comprend un solvant organique.

3. Composition antimicrobienne selon la revendication 2, comprenant une solution dans un solvant organique du sel d'un chélateur de métal de transition.

4. Composition antimicrobienne selon l'une quelconque des revendications précédentes, qui est une composition déodorante pour une utilisation sur le corps humain ou très proche de celui-ci.

5. Composition antimicrobienne selon l'une quelconque des revendications précédentes, **caractérisée en ce que** le cation du sel d'un chélateur est une amine protonée.

6. Composition antimicrobienne selon la revendication 5, **caractérisée en ce que** le cation du sel d'un chélateur est du 2-amino-2-méthyl-1-propanol, de la cyclohexylamine, de la diisopropanolamine ou du 2-aminobutan-1-ol.

7. Composition antimicrobienne selon l'une quelconque des revendications précédentes, **caractérisée en ce que** le cation organique est présent à un niveau suffisant pour neutraliser au moins 60 % de n'importe quels groupes acides de la forme acide de l'anion chélateur.

8. Composition antimicrobienne selon l'une quelconque des revendications précédentes, **caractérisée en ce que** le cation organique est présent à un niveau suffisant pour donner une solution aqueuse du sel d'un chélateur ayant un pH entre 6 et 8 (à une concentration molaire du sel d'un chélateur qui est égale à celle présente dans la composition).

9. Composition antimicrobienne selon l'une quelconque des revendications précédentes, **caractérisée en ce que** l'anion du sel d'un chélateur de métal de transition a une affinité pour le fer (III).

10. Composition antimicrobienne selon la revendication 8, **caractérisée en ce que** l'anion du sel d'un chélateur de métal de transition a un coefficient de liaison pour le fer (III) supérieur à 10²⁶.

11. Composition antimicrobienne selon l'une quelconque des revendications précédentes, **caractérisée en ce que** le sel d'un chélateur de métal alcalin est un sel d'acide polyaminocarboxylique.

12. Composition antimicrobienne selon l'une quelconque des revendications précédentes, **caractérisée en ce que** l'anion du sel d'un chélateur de métal alcalin a une forme acide comprenant au moins cinq groupes acides.

13. Composition antimicrobienne selon la revendication 12, **caractérisée en ce que** le sel d'un chélateur de métal alcalin est un sel d'acide diéthylène triamine penta acétique.

14. Composition antimicrobienne selon l'une quelconque des revendications précédentes, **caractérisée en ce que** moins de 50 % en poids d'eau est présente dans la composition, à l'exclusion de tout propulseur volatil pouvant être présent.

15. Composition antimicrobienne selon la revendication 14, **caractérisée en ce que** le ratio entre les autres composants liquides et l'eau est supérieur à 65 :35 en poids.

16. Composition antimicrobienne selon l'une quelconque des revendications précédentes, **caractérisée en ce que** le sel d'un chélateur est présent à une concentration de 0,01 % à 10 % en poids, à l'exclusion de tout propulseur volatil présent.

17. Composition antimicrobienne selon l'une quelconque des revendications précédentes, qui est sous la forme d'une composition aérosol comprenant un propulseur volatil.

18. Composition antimicrobienne aérosol selon la revendication 16, comprenant un solvant organique ayant une valeur c/logP inférieure à 2 et un propulseur volatil non chloré, ladite composition étant une composition homogène sous pression.

19. Composition antimicrobienne selon l'une quelconque des revendications précédentes, comprenant un agent antimicrobien additionnel.

20. Composition antimicrobienne selon la revendication 19, **caractérisée en ce que** l'agent antimicrobien additionnel est un bactéricide cationique.

21. Composition antimicrobienne selon la revendication 20, **caractérisée en ce que** l'agent antimicrobien additionnel est un bactéricide cationique organique.

22. Composition antimicrobienne selon l'une quelconque des revendications précédentes, comprenant un matériau de parfum à hauteur de jusque 4 % en poids de la composition.

23. Méthode cosmétique pour contrôler le nombre de microbes sur la surface externe du corps humain ou sur un vêtement porté très proche de celui-ci, ladite méthode comprenant l'application à la surface externe du corps humain ou sur un vêtement porté très proche de celui-ci, d'une composition antimicrobienne selon l'une quelconque des revendications précédentes.

24. Méthode cosmétique pour inhiber la génération d'odeurs corporelles, ladite méthode comprenant l'application à la surface externe du corps humain ou sur un vêtement porté très proche de celui-ci, d'une composition antimicrobienne selon l'une quelconque des revendications 1 à 22.

25. Méthode cosmétique pour délivrer une intensité de parfum augmentée, comprenant l'application topique à la surface externe du corps humain ou sur un vêtement porté très proche de celui-ci, d'une composition selon la revendication 22.

26. Méthode selon l'une quelconque des revendications 23 à 25, dans laquelle, dans une étape précédente, la surface externe du corps humain ou le vêtement porté très proche de celui-ci, est lavé et/ou, dans le cadre d'une étape précédente ou simultanée, est mis en contact avec un agent antimicrobien, ce qui abaisse le nombre de microbes vivants.

27. Méthode de fabrication d'une composition antimicrobienne, ladite méthode comprenant la formation d'une solution dans un solvant organique d'un sel d'un chélateur de métal de transition selon la revendication 3.

28. Méthode de fabrication d'une composition antimicrobienne selon la revendication 27, comprenant l'addition d'un chélateur acide et d'une amine dans de l'eau pour former uns solution aqueuse, suivie par la dilution avec un alcool pour former une solution aqueuse d'alcool, optionnellement suivie d'une mise sous pression avec un propulseur volatil liquéfié.
